# EUROPÄISCHE PATENTANMELDUNG

(11) **EP 2 522 312 A1**
(43) Veröffentlichungstag der Anmeldung: **14.11.2012**
(21) Anmeldenummer: 12164375.3
(22) Anmeldetag: 17.04.2012
(51) Int. Cl.: A61F 2/84, A61M 25/01

(54) **Handgerät für die Stentimplantation**

(30) Priorität: 10.05.2011 US 201161484244 P
(71) Anmelder: Biotronik AG, 8180 Bülach (CH)
(72) Erfinder: Hofmann, Eugen, 8049 Zürich (CH); Moehl, Raimund, 8127 Forch (CH)
(74) Vertreter: Lindner-Vogt, Karin L.

(57) **Zusammenfassung**

Die vorliegende Erfindung betrifft ein Handgerät (10) zur Bedienung eines Applikationssystems für die Stentimplantation mit einem Adapter (12) zum Anschluss an eine Einführschleuse (14) oder an einen Führungskatheter, durch die/den ein Draht oder Katheter in ein Gefäß einführbar ist. Der Adapter ist erfindungsgemäß als Teleskoprohr ausgeführt.

## Beschreibung

Die Erfindung betrifft ein Handgerät zur Bedienung eines Applikationssystems für die Stentimplantation mit einem Adapter zum Anschluss an eine Einführschleuse oder an einen Führungskatheters, durch die/den ein Draht oder Katheter in ein Gefäß einführbar ist.

Die Angioplastie, auch perkutane transluminale Angioplastie (PTA), ist ein Verfahren zur Erweiterung oder Wiedereröffnung von verengten oder verschlossenen Blutgefäßen zumeist mittels Ballondilatation. Bei der Ballondilatation im Rahmen einer perkutanen transluminalen Angioplastie wird ein an einem Gefäßkatheter angebrachter Ballon an der verengten Gefäßstelle langsam und unter Druck entfaltet. Dadurch wird die Engstelle so gedehnt, dass sie den Blutstrom nicht mehr oder weniger stark behindert. Die Ballonkatheter werden fast immer von der Leiste aus über einen Führungsdraht und Führungskatheter in die Stenose (Engstelle) platziert. Zusätzlich werden häufig über ein geeignetes System Stents implantiert (Stentimplantation). Ein Stent (oder auch Gefäßstütze) ist ein medizinisches Implantat, das in Hohlorgane, hier ein Blutgefäß, eingebracht wird, um sie offen zu halten. Es handelt sich meist um ein kleines Gittergerüst in Röhrchenform aus Metall oder Kunststoff.

Zur praktischen Durchführung der Stentimplantation wird in der Regel zuerst ein Führungsdraht, durch eine Einführschleuse oder einen Führungskatheter in das Gefäß eingeführt und der auf einem Trägersystem befindliche Stent über diesen Führungsdraht zum Ort der Läsion verbracht. In vielen Fällen wird die Engstelle vor dem Einführen des Stents mit Hilfe eines Ballonkatheters vorab aufgeweitet (Angioplastie). Mit der Bezeichnung Einführschleuse sind hier allgemein hämostatische Ventile gemeint. Es sind zumeist dünne Röhren aus Kunststoff, die einen Ventilmechanismus beinhalten, der den Vorschub von interventionellem Werkzeug wie Drähten und Kathetern erlaubt, dabei aber einen Austritt von Blut verhindert. Der Stent - und damit auch das Gefäß im Bereich der Läsion - wird dann am Implantationsort durch expandieren eines Ballons aufgeweitet oder, bei selbstexpandierenden Stents, durch das Zurückziehen eines Außenschafts freigesetzt.

Während des Setzen des Stents darf derselbe nicht in axialer Richtung vor oder zurück bewegt werden, da hierdurch anderenfalls eine Stauchung oder Dehnung des Stents resultieren könnte. Eine solche axiale Verformung des Stents kann zur Rissbildung oder Fraktur einzelner Segmente des Stents führen, was die Wahrscheinlichkeit für einen erneuten Gefäßverschluss (Restenose) deutlich erhöht.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung eine Stauchung oder Dehnung des Stents während der Dilatation zu verhindern.

Die vorliegende Erfindung löst diese Aufgabe durch Bereitstellung eines Handgeräts zur Bedienung eines Applikationssystems für die Stentimplantation mit einem Adapter zum Anschluss an eine Einführschleuse oder an einen Führungskatheter, durch die/den ein Draht oder Katheter in ein Gefäß einführbar ist. Der Adapter ist erfindungsgemäß als Teleskoprohr ausgeführt.

Ein Teleskoprohr besteht aus mehreren (sich ggf. zylindrisch verjüngenden und parallel) in sich geführten Röhren oder Zylindern, welche linear bis zu einem Anschlag auf eine maximale Auszugslänge herausgezogen und wieder ineinander platzsparend zurückgeschoben werden können. Das Teleskoprohr ist eine technische Verlängerung eines Armes in Röhrenform, um einen gewünschten Abstand variabel erreichen zu können. Die Lage des zu applizierenden Stents ist von Patient zu Patient variabel. Die Länge des Teleskopsrohrs im maximal ausgezogenen Zustand sollte daher so vorgegeben werden, dass das Teil des aus dem Körper des Patienten ragenden Stent-Trägersystems in eine gerade und gestreckte Position gebracht werden kann. Diese Länge setzt sich zusammen aus der Gebrauchslänge des Stent-Trägersystems abzüglich der Länge des zu implantierenden Stents und der Länge der Einführschleuse. Beträgt zum Beispiel die Gebrauchslänge vom Stent-Trägersystem 135 cm, die Stentlänge 20 cm und einer Länge der Einführschleuse 15 cm, so ergibt sich eine Länge von 100 cm. Das heißt, dass das Teleskoprohr auf mindestens 100 cm ausgezogen werden muss um eine gestreckte Position des Stent-Trägersystems gewährleisten zu können, falls der Stent direkt anschließend an das distale Ende der Einführschleuse implantiert werden soll. Je weiter im Körper der Stent implantiert werden soll, umso mehr muss das Teleskoprohr verkürzt werden.

Vorzugsweise weist das Teleskoprohr 4 bis 8 Rohre oder Zylinder auf. Die einzelnen Rohre oder Zylinder des Teleskoprohres sollten im maximal ausgefahrenen Zustand vorzugsweise noch über eine Länge von mindestens 5 cm ineinander geführt sein, um eine hinreichende Stabilität zu gewährleisten.

Der Erfindung liegt demnach der Gedanke zugrunde, dass eine unerwünschte axiale Verformung des Stents während seiner Expansion vermieden werden kann, wenn in dieser Phase des Eingriffs eine Bewegung des Handgeräts in axialer Richtung vermieden wird. Hierzu ist ein spezieller Adapter vorgesehen, der auf dem Handgerät sitzt und teleskopisch bis zur Einführschleuse oder dem Führungskatheter verlängert werden kann. Dieser Adapter nimmt die Form eines Teleskoprohres ein. Der teleskoprohrartige Adapter wird vor der Expansion des Stents auf die benötigte Länge ausgezogen, so dass das Handgerät in gerader und gestreckter Position mit der Einführschleuse oder dem Führungskatheter verbunden ist. So kann gewährleistet werden, dass es nicht zu einer unerwünschten Stauchung oder Streckung des Stents kommt. Der Adapter kann fest auf dem Handgerät fixiert oder als ein separierbares Zusatzteil ausgelegt sein. Der Adapter kann aus Metall bestehen, zum Beispiel aus chirurgischem Stahl.

Vorzugsweise weist der Adapter Arretierungsmittel auf, mit denen eine Länge des Teleskoprohes fixierbar ist. Wenn der Adapter demnach auf die gewünschte Länge ausgezogen ist, können die einzelnen Rohre, zum Beispiel durch eine Rotationsdrehung, arretiert werden. Geeignete Arretierungsmittel umfassen komplementäre Kerben und Wulste auf den Rohren oder Klemmmittel, wie Hebel oder dergleichen.

Ein weiterer Aspekt der Erfindung liegt in der Bereitstellung eines Applikationssystem für die Stentimplantation, das das zuvor beschriebene Handgerät umfasst.

Die Erfindung wird nachfolgend anhand von Ausführungsbeispielen und den dazugehörigen Figuren näher erläutert. Es zeigen:
- Fig. 1: zeigt Bestandteile eines Applikationssystems für die Stentimplantation, das ein Handgerät mit einem erfindungsgemäßen Adapter umfasst.
- Fig. 2: zeigt den erfindungsgemäßen Adapter in vergrößerter Darstellung.
- Fig. 3: dient zur Illustration der Anwendung des erfindungsgemäßen Applikationssystems im Rahmen der Stentimplantation.

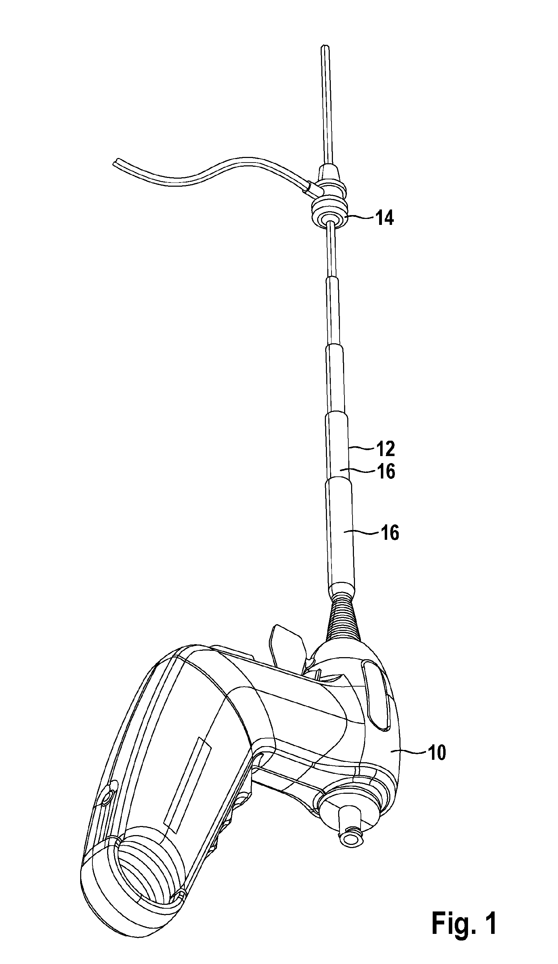

Fig. 1 zeigt ein Handgerät 10, wie es bei der Stentimplantation Einsatz findet. Das Handgerät 10 ist über einen Adapter 12 mit einer Einführschleuse 14 verbunden. Durch den Adapter 12 können im Zuge des interventionellen Eingriffs Führungsdrähte und ein Stent-Trägersystem eingeführt werden.

Der Adapter 12 zeichnet sich dadurch aus, dass er als Teleskoprohr ausgeführt ist, d.h. er besteht vorliegend aus mehreren sich zylindrisch verjüngenden und parallel in sich geführten Rohren 16, welche linear bis zu einem Anschlag auf eine maximale Auszugslänge herausgezogen und wieder ineinander platzsparend zurückgeschoben werden können. Der Adapter 12 kann beispielweise aus medizinischem Stahl bestehen.

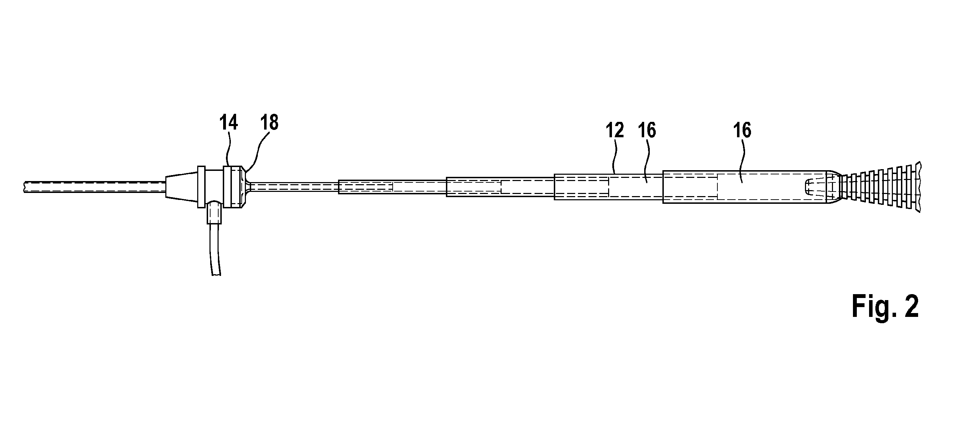

Fig. 2 zeigt den Adapter 12 nochmals in vergrößerter Darstellung. Die einzelnen Rohre 16 sind hier beispielhaft um etwa ein Drittel der maximalen Auszugslänge eingefahren. Der Adapter 12 weist ein zum Festsitz an der Einführschleuse 14 komplementär gestaltetes Ende 18 auf.

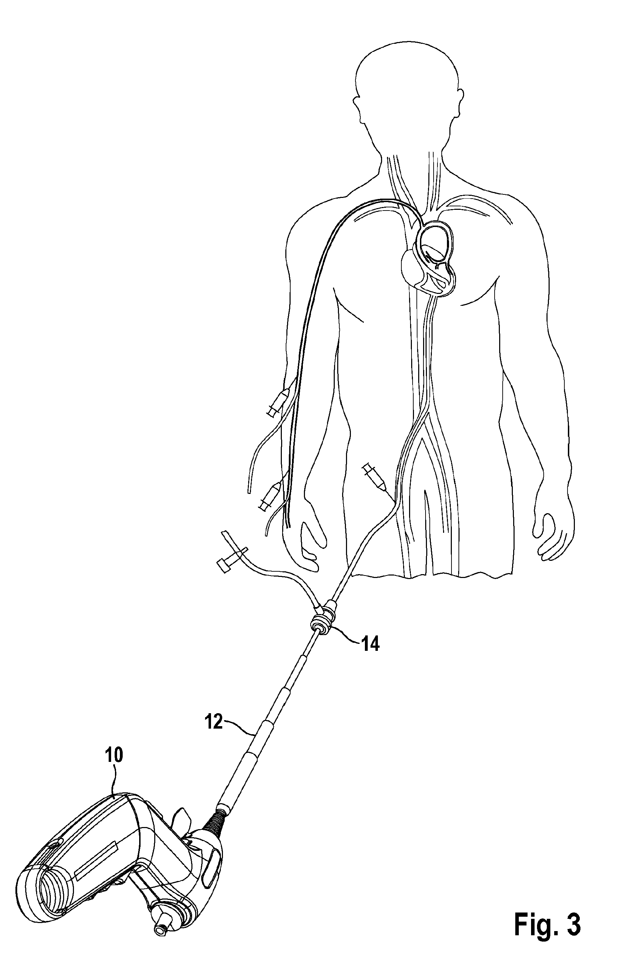

Fig. 3 illustriert schematisch die Anwendung des Applikationssystems im Rahmen der Stentimplantation. Nach Gefäßpunktierung im Lendenbereich wird eine Einführschleuse 14 gesetzt, an die das Handgerät 10 mit dem teleskoprohrartigen Adapter 12 angeschlossen wird. Im Rahmen einer perkutanen transluminalen koronaren Angioplastie (PTCA) wird unter anderem ein Stent-Applikationssystem durch die Einführschleuse 14 bis zum Herzen geführt. Im Bereich der Stenose wird ein Außenschlauch vom Stent-Applikationssystem zurückgezogen und damit gleichzeitig der Stent freigesetzt. Vor diesem Schritt wird der Adapter 12 soweit ausgefahren, dass eine axiale Bewegung des Handgeräts 10 zunächst nur zu einer Längenänderung des Adapters 12 führt, so dass eine Stauchung oder Streckung des Stents in dieser Phase vermieden werden kann.

## Patentansprüche

1. Handgerät zur Bedienung eines Applikationssystems für die Stentimplantation mit einem Adapter zum Anschluss an eine Einführschleuse oder an einen Führungskatheter, durch die/den ein Draht oder Katheter in ein Gefäß einführbar ist, wobei der Adapter als Teleskoprohr ausgeführt ist.

2. Handgerät nach Anspruch 1, bei dem das Teleskoprohr 4 bis 8 Rohre oder Zylinder aufweist.

3. Handgerät nach Anspruch 1, bei dem die einzelnen Rohre oder Zylinder des Teleskoprohres im maximal ausgefahrenen Zustand noch über eine Länge von mindestens 5 cm ineinander geführt sind.

4. Handgerät nach Anspruch 1, bei dem der Adapter Arretierungsmittel aufweist, mit denen eine Länge des Teleskoprohes fixierbar ist.

5. Applikationssystem für die Stentimplantation, umfassend ein Handgerät nach einem der Ansprüche 1 bis 4.
